# EUROPEAN PATENT SPECIFICATION

(11) **EP 1 810 615 B1**
(45) Date of publication and mention of the grant of the patent: **24.09.2008**
(21) Application number: 07250215.6
(22) Date of filing: 19.01.2007
(51) Int. Cl.: A61B 5/151

(54) **Lancing device with dampened spring**
Lanzettenvorrichtung mit gedämpfter Feder
Dispositif de lancement avec ressort mouillé

(30) Priority: 20.01.2006 US 760497 P; 31.03.2006 US 395908
(43) Date of publication of application: 25.07.2007
(73) Proprietor: LifeScan, Inc., Milpitas, CA 95035 (US)
(72) Inventor: Aylett, Jeffrey E., Southington, Connecticut 06489 (US); Uschold, Robert, Leominster, Massachusetts 01453 (US)
(74) Representative: Tunstall, Christopher Stephen

(56) References cited:
- US-A- 6 045 567

## Description

### BACKGROUND OF THE INVENTION

1. Field of the Invention

The present invention relates, in general, to medical devices and, in particular, to lancing devices and their associated methods.

2. Description of the Related Art

Conventional lancing devices generally have a rigid housing, various operating mechanisms and a lancet that can be armed and launched so as to briefly protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A bodily fluid sample (e.g., a whole blood sample) can then be expressed from the penetrated target site for collection and analysis. Conventional lancing devices are described, foe example, in U.S. Patent No. 5,730,753 to Morita, U.S. Patent No. 6,045,567 to Taylor et al., U.S. Patent No. 6,071,250 to Douglas et al., U.S. Patent No. 6,156,051 to Schraga, U.S. Patent No. 6,197,040 to LeVaughn et al., and U.S. Patent No. 6,607,543 to Purcell et al..

Conventional lancing devices typically require a user to arm the lancing device, urge the lancing device against a target site, and then press a button or other switch to manually activate the lancing device such that a lancet within the device is launched (also referred to as "fired") towards the target site. The lancet then penetrates (e.g., lances) the target site, thereby creating an opening for the expression of a bodily fluid sample.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIG. 1 is a simplified schematic cross-sectional view of a lancet holder and depth stop configuration;
FIG. 2A is a simplified schematic cross-sectional view of a lancet holder, dampener and depth stop (prior to engagement therebetween) configuration as can be employed in lancing devices according to various exemplary embodiments of the present invention;
FIG. 2B is another simplified schematic cross-sectional view of the lancet holder, dampener and depth stop (during engagement therebetween) configuration of FIG. 2A;
FIG. 3A is a simplified schematic cross-sectional view of another lancet holder, dampener and depth stop (prior to engagement therebetween) configuration as can be employed in lancing devices according to various other exemplary embodiments of the present invention;
FIG. 3B is another simplified schematic cross-sectional views of the lancet holder, dampener and depth stop (during engagement therebetween) configuration of FIG. 3A;
FIG. 4 is a simplified graph of lancet velocity as a function of lancet position for the configurations of FIGs. 1, 2A-2B and 3A-3B;
FIG. 5 is a simplified perspective view of a compact lancing device according to an exemplary embodiment of the present invention;
FIG. 6 is a simplified perspective exploded view of the compact lancing device of FIG. 5;
FIG. 7 is a simplified cross-sectional view of the compact lancing device of FIG. 5;
FIG. 8 is a simplified cross-sectional view of the compact lancing device of FIG. 5 during use and prior to a dampener of the lancing device engaging (contacting) a depth stop of the lancing device;
FIG. 9 is another simplified cross-sectional view of the compact lancing device of FIG. 5 after the dampener has engaged (made contact with) the depth stop;
FIG. 10 is a simplified cross-sectional view of a portion of FIG. 8;
FIG. 11 is a simplified cross-sectional view of a portion of FIG. 9; and
FIG. 12 is a flow diagram illustrating a sequence of steps in a method for dampened lancing according to an exemplary embodiment of the present invention.

### DETAILED DESCRIPTION OF THE INVENTION

Lancing devices according to various embodiments of the present invention include a housing, a moveable lancet holder configured to hold a lancet, a launching mechanism, a depth stop with a depth stop interface surface, and a dampener for dampening at least one of sound and vibration during lancing. The moveable lancet holder includes a lancet holder first depth stop interface surface. The moveable lancet holder, depth stop and dampener each can be, for example, at least partially disposed within the housing. In addition, the moveable lancet holder and launching mechanism are operatively connected to lance a target site (e.g., a dermal tissue target site) with the lancet and the dampener is disposed between the lancet holder first depth stop interface surface and the depth stop interface surface. Furthermore, a forward movement of the moveable lancet holder during lancing of a target site is stopped by engagement between the lancet holder first depth stop interface surface, the dampener and the depth stop interface surface.

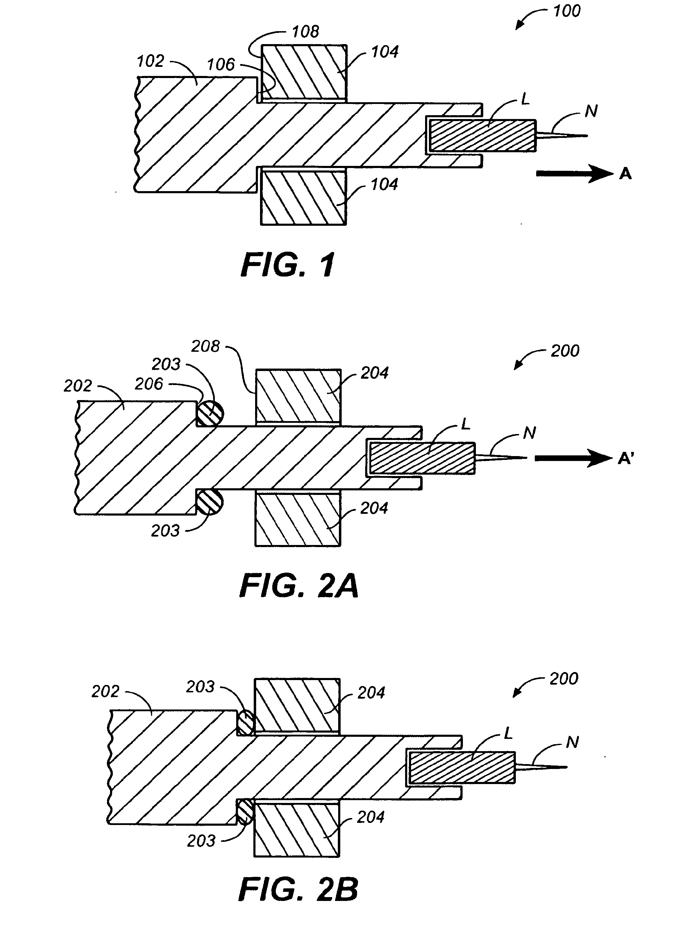

FIG. 1 is a simplified schematic cross-sectional view of configuration 100 of a lancet holder and depth stop, as could conceivably be employed in a conventional lancing device. Configuration 100 includes a moveable lancet holder 102 and a depth stop 104. Moveable lancet holder 102 is configured to hold a lancet L that includes a needle N. Moreover, moveable lancet holder 102 includes a lancet holder depth stop interface surface 106 and depth stop 104 includes a depth stop interface surface 108. Moveable lancet holder 102 can be formed, for example, of a thermoplastic elastomer such as acetal, nylon and polycarbonate-polyester blends. Depth stop 104 can be formed, for example, of a thermoplastic material such as acetal and polycarbonate.

In the configuration of FIG. 1, movement of moveable lancet holder 102 in the direction of arrow A is abruptly stopped when lancet holder depth stop interface surface 106 makes contact with (i.e., engages) depth stop interface surface 108. Such an abrupt stop can produce vibration and/or noise that are unsettling, and cause perception of pain, to a user.

FIG. 2A is a simplified schematic cross-sectional view of configuration 200 of a lancet holder, dampener and depth stop, prior to engagement therebetween, as can be employed in lancing devices according to various embodiments of the present invention. FIG. 2B is configuration 200 of a simplified schematic cross-sectional view of the lancet holder, dampener and depth stop during engagement therebetween.

Configuration 200 includes a moveable lancet holder 202, a dampener 203 and a depth stop 204. Moveable lancet holder 202 is configured to hold a lancet L that includes a needle N. Moreover, moveable lancet holder 202 includes a lancet holder depth stop interface surface 206 and depth stop 204 includes a depth stop interface surface 208.

Dampener 203 can be formed of any suitable sound and/or vibration dampening material including, for example, an elastomeric material, a copolymer of butadiene and acrylonitrile, silicone rubber, a visco-elastic polymer (e.g., a thermoset polyether-based polyurethane) or a combination thereof. Dampener 203 can be shaped, for example, as an o-ring. Suitable materials include, but are not limited to, materials with a durometer in the range of 60 to 80 on the Shore A scale.

In the configuration of FIGs. 2A and 2B, movement of moveable lancet holder 202 in the direction of arrow A' is first slowed (i.e., decelerated) and then subsequently stopped by engagement between lancet holder depth stop interface surface 206, dampener 203 and depth stop interface surface 208 (see FIG. 2B). During such an engagement, moveable lancet holder 202 is decelerated by the compression of dampener 203 between lancet holder depth stop interface 206 and depth stop interface 208 (compare FIGs. 2A and 2B). The deceleration and subsequent stopping of the moveable lancet holder in the manner described immediately above serves to reduce vibration and noise in comparison to the configuration of FIG. 1. Such a reduction in vibration and/or noise can also serve to decrease a user's perceived pain during lancing.

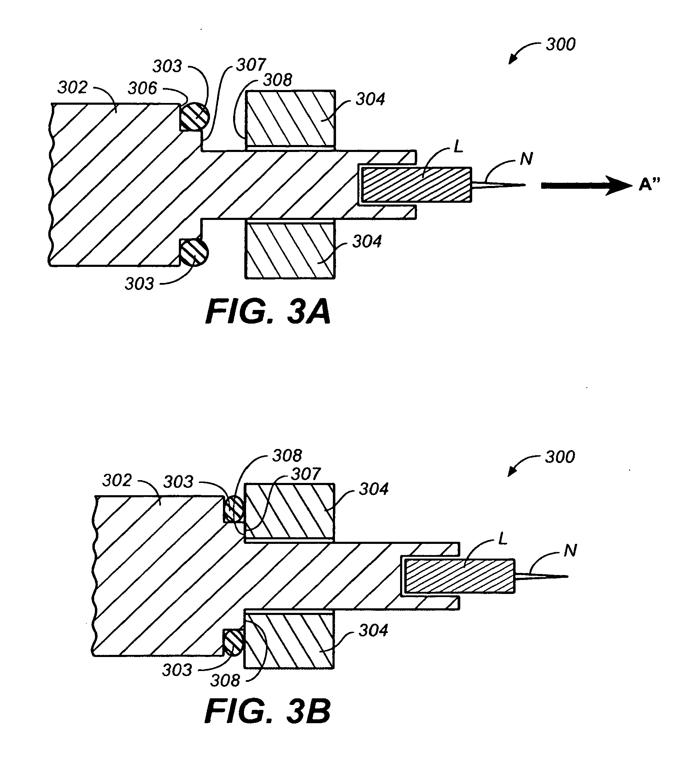

FIG. 3A is a simplified schematic cross-sectional view of configuration 300 of another lancet holder, dampener and depth stop, prior to engagement therebetween, as can be employed in lancing devices according to various other embodiments of the present invention. FIG. 3B is a simplified schematic cross-sectional view of configuration 300 of the lancet holder, dampener and depth stop during engagement therebetween.

Configuration 300 includes a moveable lancet holder 302, a dampener 303 and a depth stop 304. Moveable lancet holder 302 is configured to hold a lancet L that includes a needle N. Moreover, moveable lancet holder 302 includes a lancet holder first depth stop interface surface 306 and a lancet holder second depth stop interface surface 307. Also, depth stop 304 includes a depth stop interface surface 308.

In the configuration of FIGs. 3A and 3B, movement of moveable lancet holder 302 in the direction of arrow A" is first slowed (i.e., decelerated) by the compression of dampener 303 between lancet holder first depth stop interface surface 306 and depth stop interface surface 308. The movement of moveable lancet holder 302 in the direction of arrow A" is subsequently stopped by engagement between lancet holder second depth stop interface surface 307 and depth stop interface surface 308 (see FIG. 3B).

The deceleration and subsequent stopping of the moveable lancet holder in the manner described immediately above serves to reduce vibration and noise in comparison to the configuration of FIG. 1. Such a reduction in vibration and/or noise can also serve to decrease a user's perceived pain during lancing.

In the configuration of FIGs. 2A and 2B, the movement of moveable lancet holder 202 in the direction of arrow A' is ultimately stopped by the compression of dampener 203. Therefore, if the compressive characteristics of dampener 203 vary due to, for example, age or manufacturing tolerances, the point at which moveable lancet holder 202 is stopped will also vary. However, in the configuration of FIGs. 3A and 3B, the movement of moveable lancet holder 302 in the direction of arrow A" is ultimately stopped by direct contact between lancet holder second depth stop interface surface 307 and depth stop interface 308, thus eliminating the potential for variability in the point at which moveable lancet holder 302 is stopped. Despite this characteristic of configuration 300, configuration 200 may be preferred since the ultimate stopping of moveable lancet holder 202 solely by compression of dampener 203 can result in less noise than the ultimate stopping of moveable lancet holder 302 by contact between lancet holder second depth stop interface surface 307 and depth stop interface 308.

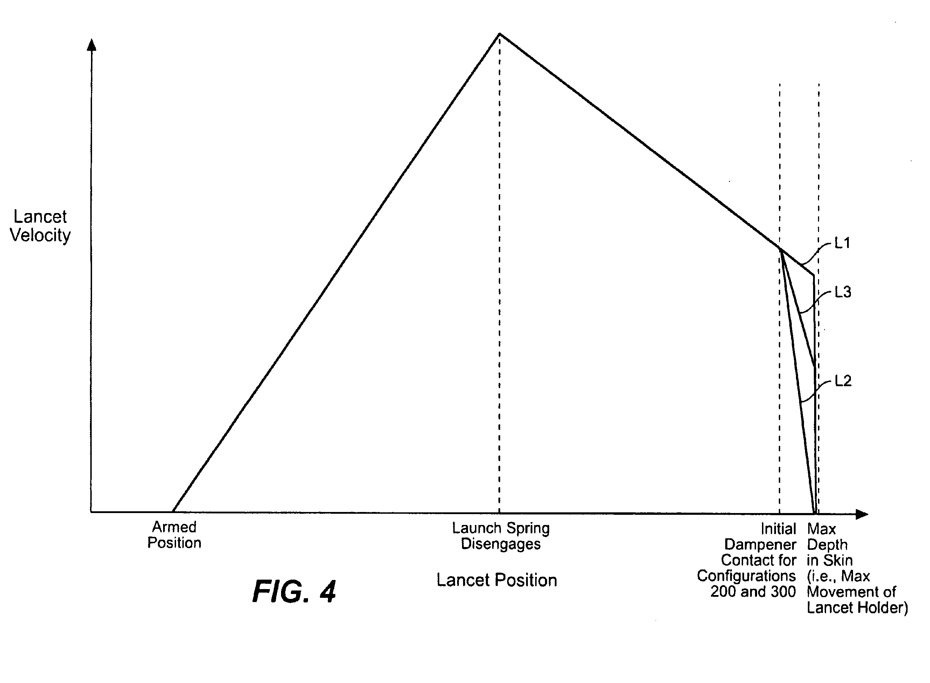

FIG. 4 is a simplified graph of relative lancet velocity as a function of lancet position for the configurations of FIGs. 1, 2A-2B and 3A-3B. FIG. 4 is hypothetical in nature and not necessarily to scale and is intended for descriptive and explanatory purposes. Line L1 represents lancet velocity in the absence of a dampener (i.e., configuration 100 of FIG. 1). Line L2 represent lancet velocity for configuration 200 and line L3 represents lancet velocity for configuration 300.

Configuration 100 results in lancet velocity dropping essentially instantaneously to zero when lancet holder depth stop interface surface 106 engages directly with depth stop interface surface 108 (see line L1 of FIG. 4). For configuration 200 (line L2), dampener 203 is compressed, causing deceleration of the moveable lancet holder until the movement of the moveable lancet holder is stopped. For configuration 300 (line L3), contact of dampener 303 with depth stop interface surface 308 causes deceleration of moveable lancet holder 302 until lancet holder second depth stop interface surface 307 makes contact with depth stop interface surface 308 (see FIG. 3B), at which time the lancet velocity rapidly approaches zero. As previously noted, the deceleration of the moveable lancet holder prior to its stopping serves to beneficially reduce vibration and/or noise during lancing. A typical, but non-limiting, duration for the deceleration of configurations 200 and 300 is approximately 300 micro-seconds.

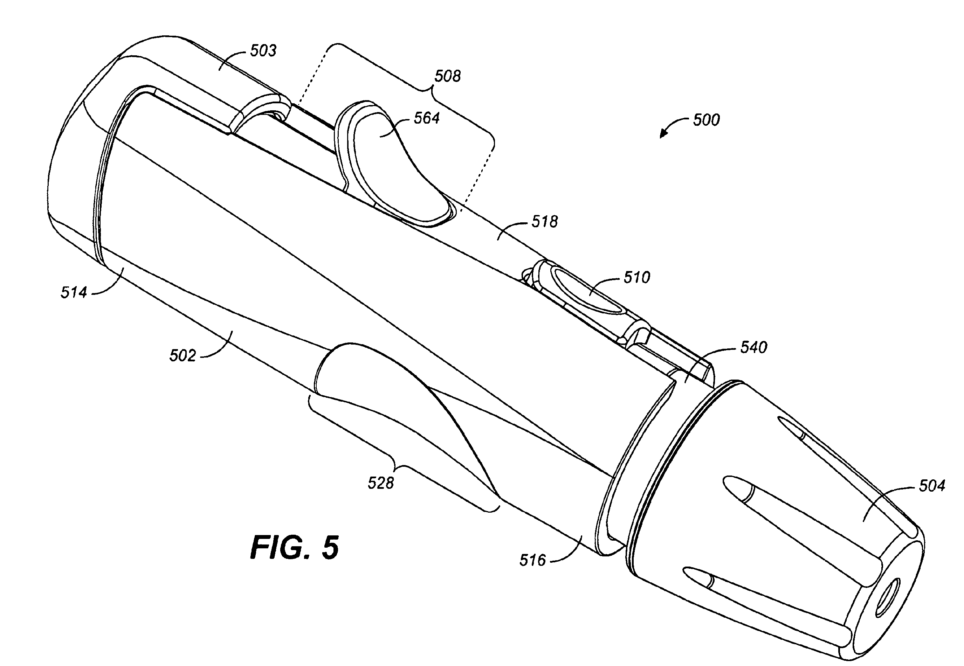

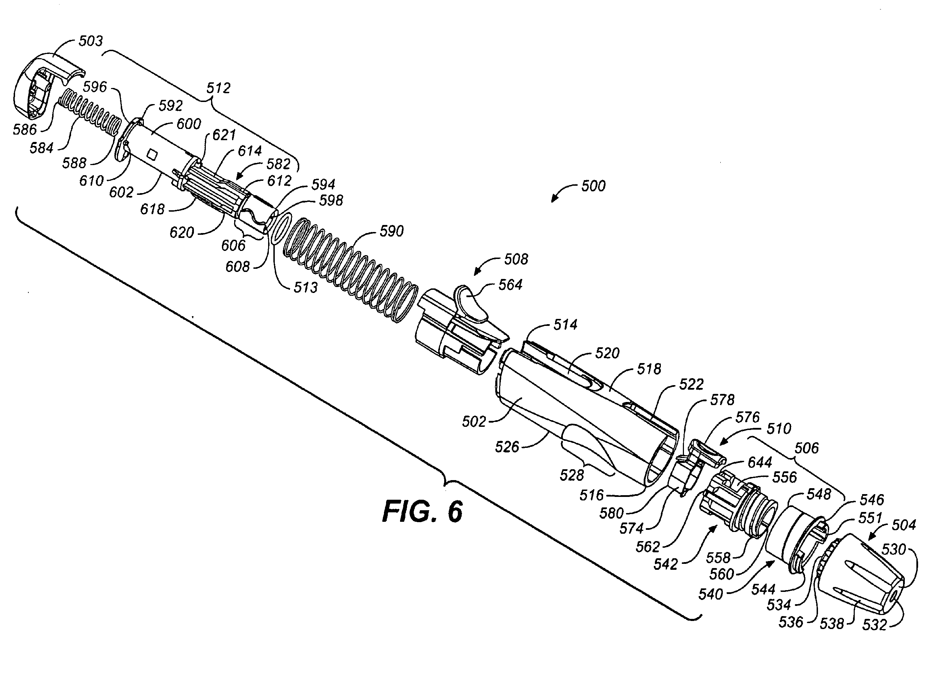

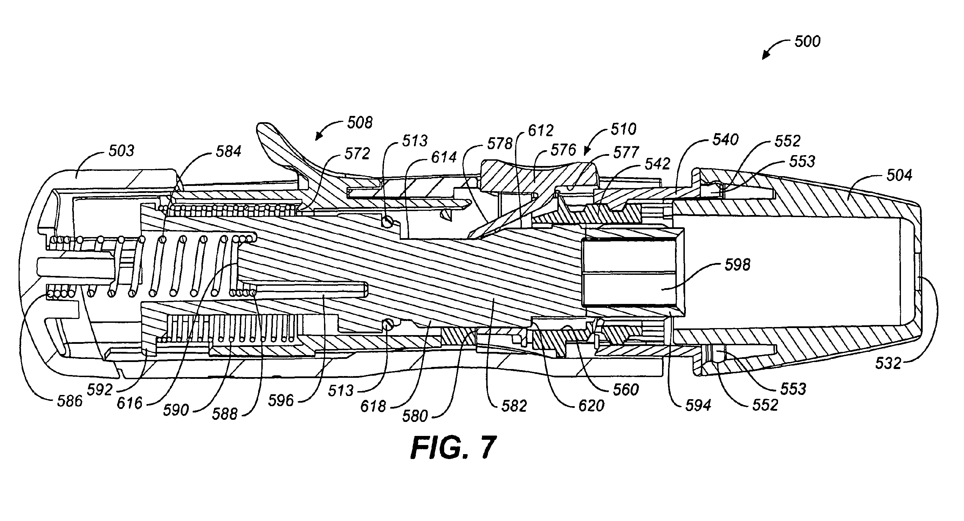

FIGs. 5, 6 and 7 are various simplified depictions of a compact lancing device 500 according to an exemplary embodiment of the present invention. FIG. 5 is a simplified perspective view of compact lancing device 500. FIG. 6 is a simplified perspective exploded view of compact lancing device 500 and FIG. 7 is a simplified cross-sectional view of compact lancing device 500.

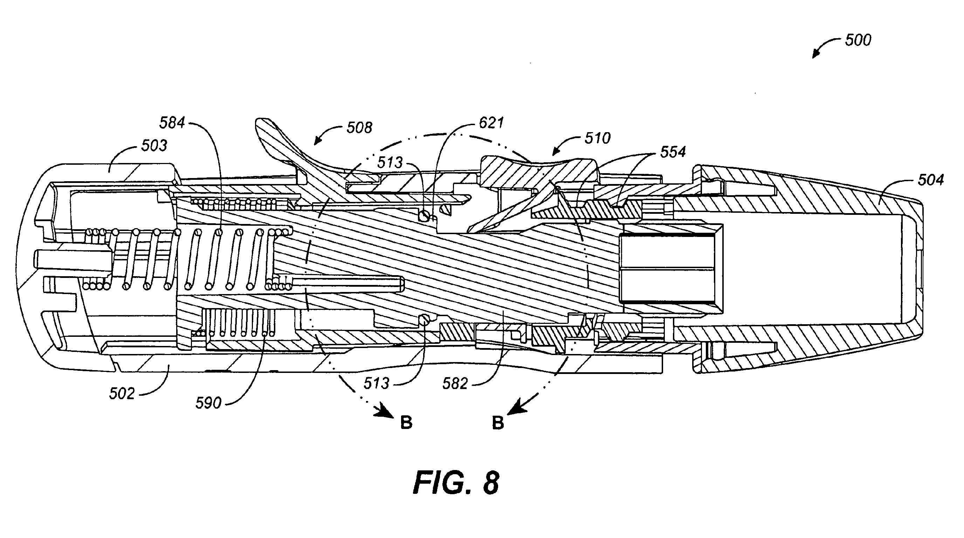

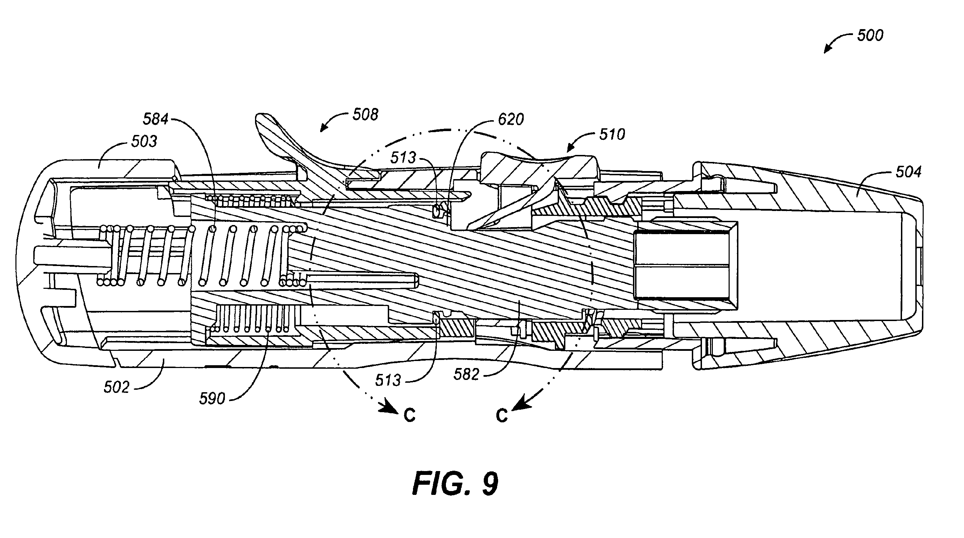

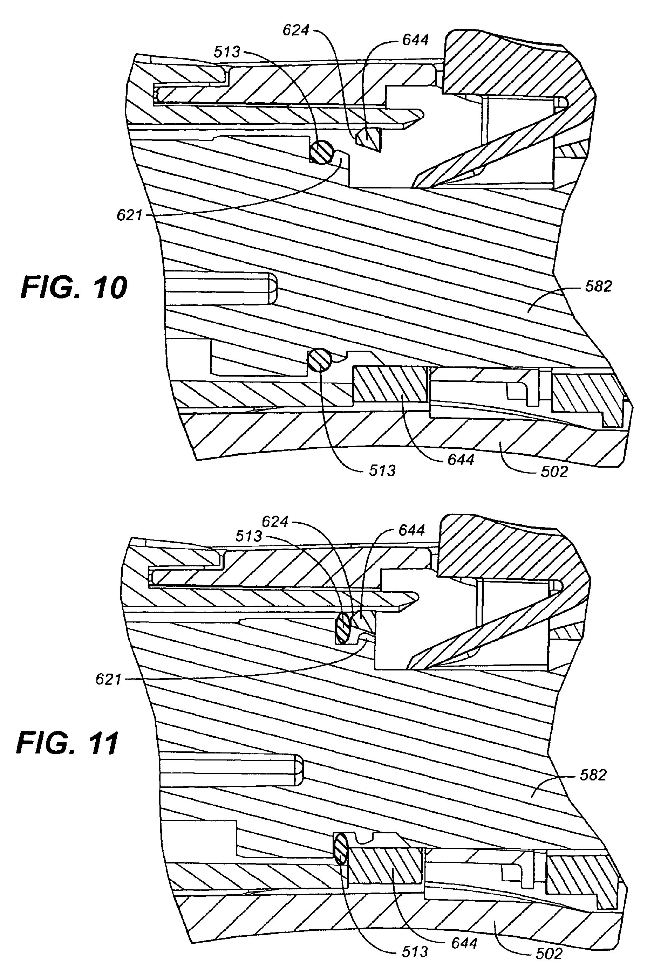

FIG. 8 is a simplified cross-sectional view of compact lancing device 500 during use prior to a dampener of the lancing device engaging (contacting) a depth stop of the lancing device. FIG. 9 is another simplified cross-sectional view of the compact lancing device of FIG. 1 after the dampener has engaged (made contact with) the depth stop. FIG. 10 is a simplified cross-sectional view of a portion of FIG. 8 from within hashed double-headed arrow B-B of FIG. 8. FIG. 11 is a simplified cross-sectional view of a portion of FIG. 9 from within hashed double-headed arrow C-C of FIG. 9.

Referring to FIGs. 5 through 11, compact lancing device 500 includes a housing 502, an end cap 503, a finger cap 504, a depth adjustment mechanism 506 (see FIG. 6 in particular), an arming mechanism 508, a trigger mechanism 510, a launching mechanism 512 and a dampener 513.

As described in detail below, launching mechanism 512, arming mechanism 508 and trigger mechanism 510 are operatively connected such that a target site (e.g., a user's dermal tissue target site) can be lanced with a lancet (not shown in FIGs. 5 through 11) held within compact lancing device 500. In this regard, launching mechanism 512 is configured for launching a lancet such that a needle of the lancet lances a target site, while arming mechanism 508 is configured for arming compact lancing device 500 prior to firing the lancing device (i.e., prior to launching the lancet), and trigger mechanism 510 is configured to actuate the firing of compact lancing device 500. Furthermore, depth adjustment mechanism 506 is configured for a user to select (i.e., predetermine) needle penetration depth into the target site.

Compact lancing device 500 can be any suitable size but can be beneficially sized to fit within the palm of a user's hand and has, therefore, a typical but non-limiting length in the range of 70 mm to 90 mm and a typical but non-limiting width in the range of about 10 mm to about 20 mm. Such a compact size is beneficial in that it requires less storage space and is less conspicuous than conventionally sized lancing devices.

Housing 502 is generally cylindrical in shape and includes a proximal end 514, a distal end 516, a first surface 518, an arming mechanism orifice 520, a trigger mechanism orifice 522, a second surface 526 and a gripping feature 528. Second surface 526 may be, for example, flat to prevent compact lancing device 500 from rolling when placed on a surface.

Housing 502 can be formed, for example, of rigid materials including, but not limited to, polycarbonate, polyester, polystyrene, polyamide, polyacetal, polyimide, polyketone, polyurethane, polybutyleneteraphthalate and combinations thereof. Housing 502 can also be formed of semi-rigid materials including, for example, polypropylene, high-density polyethylene, polyurethane, ethylene propylene rubber, polymethylpentene and combinations thereof. If desired, housing 502 can be easily manufactured from two elongate pieces that are glued, ultrasonically welded or snap-fit together to create housing 502. Proximal end 514 of housing 502 is closed with end cap 503, which may also be attached using, for example, glue, ultrasonic welding or snap-fit features.

Finger cap 504 is detachably connected to collar 540. Finger cap 504 includes dermal tissue (i.e., skin) engaging surface 530 with opening 532 therein (through which a lancet needle passes during lancing), collar engaging end 534 with raised features 536 and indentations 538.

Depth adjustment mechanism 506 includes a collar 540 and a guide member 542. Collar 540 includes a collar first end 544 with collar rim 546, a collar second end 548, depth setting indicator (not depicted), recesses 551 and internal spiral thread 554. Furthermore, guide member 542 includes an aperture 556, external spiral thread 558, a guide member groove 560 and outer protrusions 562. Depth adjustment mechanism 506 also includes a depth stop interface surface (element 624 described below) on a depth stop 644.

Arming mechanism 508 includes a handle 564, an internal groove (not shown) and an internal raised portion (also not shown). Trigger mechanism 510 includes a body 574, a trigger button 576, a spring element 578 and a latch rim 580.

Launching mechanism 512 includes a moveable lancet holder 582, a launch spring 584 (with launch spring first and second ends 586 and 588, respectively) and a retraction spring 590.

Moveable lancet holder 582 includes proximal end 592, a distal end 594, a first hollow portion 596, a second hollow portion 598, a first surface 600, a second surface 602., a radially and outwardly expandable portion 606, a slit 608, a retaining features 610, a cam surface 612, a depression 614, an internal surface 616 and an elongate projection 618 (with a projection end 620). Moveable lancet holder 582 also includes projections 621 that retain dampener 513 (see FIGs. 8, 9, 10 and 11 in particular).

Having introduced the majority of the components of compact lancing device 500, details of the interaction and functioning of such components will now be described with reference to FIGs. 5 through 11. A portion of arming mechanism 508 is visible to a user through arming mechanism orifice 520 of housing 502. Handle 564 of arming mechanism 508 protrudes through arming mechanism orifice 520 near proximal end 514 of housing 502 and on the housing's first surface 518. A user slides handle 564 proximally to arm compact lancing device 500.

Trigger mechanism 510 is accessible to a user through trigger member orifice 522 on first surface 518 of housing 502. Trigger member orifice 522 is in close proximity to, and on the same housing surface (i.e., housing's first surface 518) as, housing's arming mechanism orifice 520 in order that a user can operate both trigger mechanism 510 and arming mechanism 508 using one hand.

A depth setting indicator (not shown) for lancing is visible to a user through trigger member orifice 522 near distal end 516 on first surface 518 of housing 502. In addition, housing 502 includes a gripping feature 528 on second surface 526 of housing 502. Second surface 526 and gripping feature 528 are in oppositional relationship to handle 564 and trigger button 576 such that a user can easily grip and operate compact lancing device 500 with one hand.

In the embodiment of compact lancing device 500, gripping feature 528 is an indentation in second surface 526 of housing 502. However, once apprised of the present disclosure, those skilled in the art will recognize that gripping feature 528 can take any suitable form, shape or texture (and can be formed of any suitable material) including, but not limited to, one or more protrusions or recesses on the surface of housing 502.

Moveable lancet holder 582 is generally cylindrical in shape with first hollow portion 596 and second hollow portion 598 disposed at the proximal and distal ends 592 and 594, respectively, of moveable lancet holder 582 (see, for example, FIG. 7). First hollow portion 596 extends into moveable lancet holder 582 approximately a third of the distance from proximal end 592 to distal end 594. Second hollow portion 598 extends into moveable lancet holder 582 approximately a third of the distance from distal end 594 to proximal end 592. Launch spring 584 is located at least partially within first hollow portion 596. Furthermore, moveable lancet holder 582 is configured such that a lancet (not shown and that includes a needle) can be removably retained at least partially within second hollow portion 598.

Distal end 594 includes a radially and outwardly expandable portion 606 with a slit 608 configured such that lancet (e.g., a suitable commercially available lancet) can easily be inserted into and removed from moveable lancet holder 582.

Proximal end 592 includes a retaining features 610 that holds retraction spring 590 in surrounding relationship to proximal end 592 of moveable lancet holder 582. First surface 600 includes a cam surface 612, adjacent to a depression 614, for cooperation with (e.g., to react against) a spring element 578 of trigger mechanism 510. Second surface 602 includes an elongate projection 618 that, along with cam surface 612 and depression 614, function during arming and triggering operations, as will be described below.

Launch spring 584 is configured to control movement of moveable lancet holder 582. Launch spring first end 586 engages an internal surface of end cap 503, while launch spring second end 588 engages an internal surface 616 of moveable lancet holder 582 (see, for example, FIG. 7). Launch spring 584 typically applies a spring force to moveable lancet holder 582 during launch of a lancet in the range of from about 0.25 pounds to 2 pounds and preferably from about 0.5 pounds to 1 pounds. In the embodiment of compact lancing device 500, retraction spring 590 is essentially concentric with launch spring 584, thereby contributing to the compactness of compact lancing device 500.

Retraction spring 590 resides substantially within the circumferential space between arming mechanism 508 and moveable lancet holder 582. Retraction spring 590 pulls moveable lancet holder 582 back after a lancet has been launched into a target site, dampens vibrations from moveable lancet holder 582 during use of compact lancing device 500 and prevents a lancet needle from penetrating the target site a second time. Retraction spring 590 also returns arming mechanism 508 to a rest position after latching. One end of retraction spring 590 is also engaged by arming mechanism 508 during arming of compact lancing device 500. Retraction spring 590 can be formed from any suitable material including plastic materials (such as polypropylene and polyester), metal materials or any combinations thereof.

Launch spring 584 and/or retraction spring 590 are/is coated with a dampening material that aides in dampening at least one of sound and vibration during lancing. Such spring coating materials may include, for example, a polymeric material such as Teflon, silicone, nylon or any combination thereof. Launch spring 584 and retraction spring 590 may be completely or partially coated at a thickness, for example, in the range of about 0.005 millimeters to about 0.015 millimeters by processes known to those skilled in the art such as, for example, dip or spray coating either before or after forming the turns in the springs. Launch and retraction springs that have been at least partially coated with a dampening material are hereinafter referred to as dampened springs.

Arming mechanism 508 is generally hollow and elongate and is disposed in surrounding relationship to moveable lancet holder 582. Internal raised portion 572 of arming mechanism 508 engages one end of retraction spring 590 during arming of compact lancing device 500, as is described below.

Elongate projection 618 of moveable lancet holder 582 is adapted to slidably move within an internal groove 560 in guide member 542 that engages elongate projection 618. The internal groove, therefore, limits relative rotational motion of moveable lancet holder 582 during use of compact lancing device 500, thereby reducing vibration and/or pain perceived by a user.

Trigger mechanism 510 is generally internally elongate, ring-shaped and disposed in a surrounding relationship to moveable lancet holder 582. Trigger mechanism 510 can move laterally but not longitudinally relative to housing 502.

Spring element 578 projects inwardly from an inner surface 577 of trigger button 576. Spring element 578 engages cam surface 612 when compact lancing device 500 is armed (not shown) and slidably engages depression 614 when a lancet is fired as moveable lancet holder 582 moves toward distal end 516 of housing 502. In both the armed and fired position of moveable lancet holder 582, spring element 578 is at a minimal load while retaining an armed or loaded position, but is momentarily loaded to a greater extent when trigger button 576 is pressed to unlatch moveable lancet holder 582. Therefore, the typical load on spring element 578 is low (e.g., less than 20 grams) even when compact lancing device 500 is armed, thus improving the durability of compact lancing device 500. Trigger mechanism 510 can be formed (e.g., molded) in one piece, thus reducing the number of components and simplifying the manufacture of compact lancing device 500.

When compact lancing device 500 is armed, latch rim 580 of trigger mechanism 510 engages projection end 620 of elongate projection 618 and trigger button 576 moves laterally to a triggering position. When a lancet is fired (i.e., when trigger button 576 is depressed), latch rim 580 slides over elongate projection 618, allowing moveable lancet holder 582 to move toward distal end 516 of housing 502.

Depth adjustment mechanism 506 enables a user to predetermine a depth of needle penetration into a target site. Finger cap 504 includes opening 532 for a lancet needle to pass through and a plurality of indentations 538 such that a user can grip finger cap 504 and rotate, tip or pull the end cap away from housing 502 when replacing lancets. Finger cap 504 can be formed of any suitable material including, but not limited to, partially flexible polymers such as polycarbonate or ABS, or elastomeric materials such as rubber, latex or silicone such that when finger cap 504 is removed, finger cap 504 can optionally deform inward and grab onto a lancet, thereby allowing a lancet to be removed along with finger cap 504.

Collar engaging end 534 of finger cap 504 is configured to mate with collar first end 544. Collar engaging end 534 includes a plurality of raised features 536 for engaging with a plurality of corresponding recesses 551 of collar 540. Raised features 536 and recesses 551 provide torque transmission from finger cap 504 to collar 540 so that the user may rotate the finger cap to adjust the depth setting. An undercut 552 on inner proximal end of finger cap 504 and a plurality of protrusions 553 on distal end of collar 540 engage with slight mechanical interference to provide for detent-based retention of finger cap 504, yet facilitate easy removal of finger cap 504 by, for example, tipping to one side or pulling off.

Collar 540 includes an internal spiral thread 554 that engages a corresponding external spiral thread 558 (which is essentially an external spiral thread cam surface) on guide member 542, a collar rim 546 on collar first end 544 and a collar second end 548. Collar 540 can rotate and slide relative to housing 502 and guide member 542 and has a generally hollow cylindrical shape.

Guide member 542 is held stationary relative to moveable lancet holder 582 by attaching guide member 542 to the inner surface of housing 502 via outer protrusions 562 that mate with recesses (not shown) on the inner surface of housing 502. However, any attachment means known to those skilled in the art can be used to secure guide member 542 to housing 502 including, but not limited to a pin, a screw, adhesives and ultrasonic welding.

Rotation of finger cap 504 adjusts the depth of needle penetration. When finger cap 504 is rotated, raised features 536 engage with collar's recesses 551 via a spline interface methodology. This causes internal spiral threads 554 of collar 540 to engage external spiral thread 558 of guide member 542, thereby moving finger cap 504 away from or toward housing 502 and changing the distance a needle penetrates into a target site.

Arming mechanism 508. trigger mechanism 510, moveable lancet holder 582, collar 540 and guide member 542 can, for example, be formed of rigid materials including, but not limited to, polycarbonate, polyester, polystyrene, polyamide, polyacetal, polyimide, polyketone, polyurethane polybutyleneteraphthalate or combinations thereof. Arming mechanism 508, trigger mechanism 510, moveable lancet holder 582, collar 540 and guide member 542 can optionally contain lubricating additives including, for example, silicone oil, Teflon or graphite to reduce friction (and resulting friction, wear and vibration) therebetween.

Referring in particular to FIGs. 8, 9, 10 and 11, during use of compact lancing device 500, and after a lancet has been launched, the forward motion of moveable lancet holder 582 is stopped by engagement of dampener 513 with depth stop interface surface 624. Dampener 513 slows the velocity of the lancet (i.e., decelerates the lancet) until the velocity is zero, thus advantageously reducing the vibration and/or noise produced such that the user perceives less pain.

Dampener 513 can be formed of, for example, an elastomeric material, a copolymer of butadiene and acrylonitrile, silicone rubber, Sorbothane or any combination thereof and can be formed as 0-ring.

In the embodiment shown in FIGs. 5 through 11, the depth stop interface is illustrated as a component of depth adjustment mechanism 506 and the dampener is illustrated as being retained on moveable lancet holder 582. However, as should be understood to those skilled in the art, the depth stop interface surface and dampener can be disposed on another suitable surface of the lancing device such as, for example, the housing or arming handle.

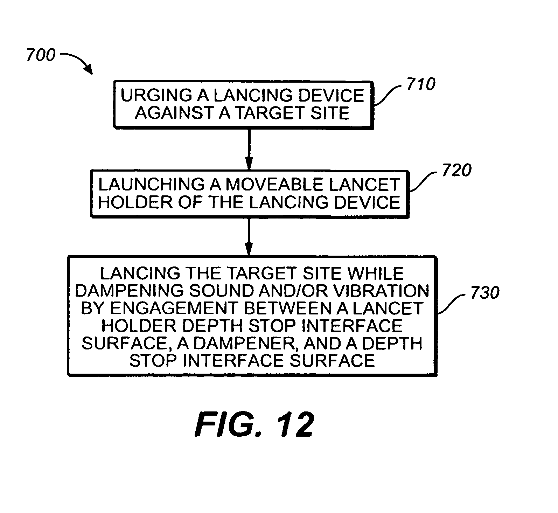

FIG. 12 is a flow diagram illustrating a method 700 for dampened lancing according to an exemplary embodiment of the present invention. Method 700 includes urging a lancing device against a target site, e.g., a dermal tissue target site, as set forth in step 710.

Subsequently, launching a moveable lancet holder of the lancing device is launched such that the moveable lancet holder moves toward the target site, as set forth in step 720. The target site is then lanced with a lancet held by the moveable lancet holder while dampening of at least one of sound and vibration occurs by engagement between a lancet holder depth stop interface, dampener and depth stop interface surface of the lancing device (refer to step 730).

Once apprised of the present disclosure, one skilled in the art will recognize that methods according to the present invention, including method 700, can be accomplished using lancing devices according to the present invention including, but not limited to the lancing device of FIGs. 5 through 11 and lancing devices employing the configurations of FIGs. 2A, 2B, 3A and 3B. In addition and if desired, any of the beneficial characteristics and operating features of such lancing devices can be incorporated in methods according to the present invention including, for example, method 700.

## Claims

1. A lancing device comprising:
a housing (502);
a moveable lancet holder (582) disposed at least partially within the housing and configured to hold a lancet; and
a launching mechanism (512) that includes a launch spring (584) and a retraction spring (590), with at least one of the launch spring (584) and retraction spring (590) being at least partially coated with a dampening material;
wherein the moveable lancet holder (582) and launching mechanism (512) are operatively connected to lance a target site with the lancet.

2. The lancing device of claim 1, wherein the launch spring has been at least partially coated with the dampening material to a thickness in the range of about 0.005 mm to about 0.015 mm.

3. The lancing device of claim 2, wherein the dampening material is a polymeric material.

4. The lancing device of claim 1, wherein the retraction spring has been at least partially coated with the dampening material to a thickness in the range of about 0.005mm to about 0.015mm.

5. The lancing device of claim 4, wherein the dampening material is a polymeric material.

6. The lancing device of claim 1, wherein the retraction spring and the launch spring are coated with the dampening material to a thickness in the range of about 0.005mm to about 0.015mm.

7. The lancing device of claim 6, wherein the dampening material is a polymeric material.

## Patentansprüche

1. Stechvorrichtung, umfassend:
ein Gehäuse (502);
einen beweglichen Lanzettenhalter (582), der zumindest partiell innerhalb des Gehäuses angeordnet und zum Halten einer Lanzette eingerichtet ist; und
einen Abschussmechanismus (512), der eine Abschussfeder (584) und eine Rückzugfeder (590) umfasst, wobei zumindest eine der Abschussfeder (584) und der Rückzugfeder (590) zumindest partiell mit einem Dämpfungsmaterial beschichtet ist;
wobei der bewegliche Lanzettenhalter (582) und der Abschussmechanismus (512) operativ verbunden sind, um mit der Lanzette in eine Zielstelle zu stechen.

2. Stechvorrichtung nach Anspruch 1, wobei die Abschussfeder zumindest partiell mit dem Dämpfungsmaterial bis zu einer Dicke im Bereich von ungefähr 0,005 mm bis ungefähr 0,015 mm beschichtet wurde.

3. Stechvorrichtung nach Anspruch 2, wobei das Dämpfungsmaterial ein Polymermaterial ist.

4. Stechvorrichtung nach Anspruch 1, wobei die Rückzugfeder zumindest partiell mit dem Dämpfungsmaterial bis zu einer Dicke im Bereich von ungefähr 0,005 mm bis ungefähr 0,015 mm beschichtet wurde.

5. Stechvorrichtung nach Anspruch 4, wobei das Dämpfungsmaterial ein Polymermaterial ist.

6. Stechvorrichtung nach Anspruch 1, wobei die Rückzugfeder und die Abschussfeder mit dem Dämpfungsmaterial bis zu einer Dicke im Bereich von ungefähr 0,005 mm bis ungefähr 0,015 mm beschichtet sind.

7. Stechvorrichtung nach Anspruch 6, wobei das Dämpfungsmaterial ein Polymermaterial ist.

## Revendications

1. Dispositif de lancement comprenant :
◆ un logement (502) ;
◆ un support de lance mobile (582) disposé au moins partiellement à l'intérieur du logement et configuré pour supporter une lance ; et
◆ un mécanisme de lancement (512) comprenant un ressort de lancement (584) et un ressort de rétraction (590), au moins un élément parmi le ressort de lancement (584) et le ressort de rétraction (590) étant au moins partiellement recouvert d'un matériau d'amortissement ;
dans lequel le support de lance mobile (582) et le mécanisme de lancement (512) sont raccordés opérationnellement pour lancer la lance sur un site cible.

2. Dispositif de lancement selon la revendication 1, dans lequel le ressort de lancement est au moins partiellement recouvert du matériau d'amortissement sur une épaisseur comprise dans la plage allant d'environ 0,005 mm à environ 0,015 mm.

3. Dispositif de lancement selon la revendication 2, dans lequel le matériau d'amortissement est un matériau polymère.

4. Dispositif de lancement selon la revendication 1, dans lequel le ressort de rétraction est au moins partiellement recouvert du matériau d'amortissement sur une épaisseur comprise dans la plage allant d'environ 0,005 mm à environ 0,015 mm.

5. Dispositif de lancement selon la revendication 4, dans lequel le matériau d'amortissement est un matériau polymère.

6. Dispositif de lancement selon la revendication 1, dans lequel le ressort de rétraction et le ressort de lancement sont recouverts du matériau d'amortissement sur une épaisseur comprise dans la plage allant d'environ 0,005 mm à environ 0,015 mm.

7. Dispositif de lancement selon la revendication 6, dans lequel le matériau d'amortissement est un matériau polymère.
